# EUROPÄISCHE PATENTSCHRIFT

(11) **EP 1 266 073 B1**
(45) Veröffentlichungstag und Bekanntmachung des Hinweises auf die Patenterteilung: **14.04.2004**
(21) Anmeldenummer: 01900444.9
(22) Anmeldetag: 13.01.2001
(51) Int. Cl.: D21C 9/00, D21D 5/26, D21H 23/08

(54) **VERFAHREN ZUR BESTIMMUNG EINES KENNWERTES FÜR DAS BINDEKRÄFTE-POTENTIAL VON SUSPENDIERTEN PAPIERFASERN SOWIE VERWENDUNG DES VERFAHRENS**
METHOD FOR DETERMINING A CHARACTERISTIC VALUE FOR THE BINDING FORCE POTENTIAL OF SUSPENDED PAPER FIBERS AND USE OF THIS METHOD
PROCEDE POUR DETERMINER UN PARAMETRE POUR LE POTENTIEL DES FORCES DE LIAISON DE FIBRES DE PAPIER EN SUSPENSION, ET APPLICATION DU PROCEDE

(30) Priorität: 26.01.2000 DE 10003383
(43) Veröffentlichungstag der Anmeldung: 18.12.2002
(73) Patentinhaber: Voith Paper Patent GmbH, 89509 Heidenheim (DE)
(72) Erfinder: SCHWARZ, Michael, 89522 Heidenheim (DE)
(86) Internationale Anmeldenummer: PCT/EP2001/000385
(87) Internationale Veröffentlichungsnummer: WO 2001/055501

(56) Entgegenhaltungen:
- EP-A- 0 349 860
- WO-A-99/53137

## Beschreibung

Die Erfindung betrifft ein Verfahren gemäß dem Oberbegriff des Anspruchs 1.

Bekanntlich werden bei der Papiererzeugung Papierfasern in einer wässrigen Fasersuspension auf die Papiermaschine gebracht und entwässert. Dabei entwickeln die sich berührenden Papierfasern Bindekräfte, die letztendlich dazu führen, dass ein fest zusammenhängendes Papierblatt entsteht. Die Möglichkeit, solche Bindekräfte zu entwickeln, also das Bindekräfte-Potential, ist von einer Vielzahl von Parametern abhängig. Die wichtigsten sind Faserzustand und Fasergröße sowie die chemische und stoffliche Umgebung in der Suspension. Die Verbesserung des Bindekräfte-Potentials ist oft mit Kosten für Rohstoffqualität, Verarbeitung und Einsatz von chemischen Hilfsmitteln verbunden. Es ist daher wünschenswert, das Bindekräfte-Potential rechtzeitig ermitteln zu können, d.h. vor der eigentlichen Papierproduktion, um den Gesamtprozess optimieren zu können.

Aus der EP 349 860 A2 ist ein Verfahren bekannt, mit dem die Retentionsmittelzugabe bei der Papierherstellung gesteuert wird. Dabei wird eine Korrelation zwischen dem laufend gemessenen Flockungsgrad und charakteristischen Werten der Faserstoffsuspension (z.B. pH-Wert, Mahlgrad) ausgewertet.

Der Erfindung liegt die Aufgabe zu Grunde, ein Verfahren zu schaffen, mit dem das Bindekräfte-Potential der Papierfasern in einer wässrigen Suspension charakterisiert werden kann. Insbesondere sollen Abweichungen vom gewünschten Bindekräfte-Potential rechtzeitig feststellbar sein. Die Messung soll zu schnellen Messergebnissen führen.

Die Aufgabe wird durch die im Kennzeichen des Anspruchs 1 genannten Merkmale gelöst.

In der WO 99/53137 ist eine Möglichkeit genannt, um den Luftgehalt einer Faserstoffsuspension zu messen.

Dabei ergeben sich besonders gut verwertbare Messwerte an der Stelle, an der noch keine Maßnahmen zur wirksamen Entlüftung der Faserstoffsuspension erfolgt sind. Dort kann sich der Luftgehalt entsprechend den fasertechnologischen und chemischen Parametern des zur Papiererzeugung aufbereiteten Faserstoffes frei einstellen. Überraschenderweise lässt sich die Korrelation aus dem an dieser Stelle gemessenen Luftgehalt und dem Bindekräfte-Potential ausreichend genau definieren, so dass eine frühzeitige Aussage über bestimmte zu erwartende Papiereigenschaften getroffen werden kann. Diese Korrelation lässt sich z.B. durch empirische Messungen leicht ermitteln, auch ohne dass alle kausalen Zusammenhänge bekannt sind. Dabei kann man mit Erfahrungswerten arbeiten, die durch Messung der Festigkeiten der aus der Suspension gebildeten Blätter ermittelt werden.

Es ist anzunehmen, dass nicht der Luftgehalt selbst für das Bindekräfte-Potential von Bedeutung ist, sondern dass er nur als Indikator dient. In vielen Fällen wurde ein höheres Bindekräfte-Potential bei höherem Luftgehalt festgestellt.

In der Praxis wird die Luftgehaltsmessung z.B. im Konstanten Teil der Papierfabrik nach der Mischpumpe vorgenommen, in der der Papierstoff mit dem Verdünnungswasser vermischt und auf die erforderliche Stoffdichte gebracht wird. Wenn das Papier oder der Karton aus verschiedenen Sorten hergestellt werden soll, können die Kennwerte für diese Sorten auch einzeln ermittelt und Korrekturen individuell vorgenommen werden.

Es sind verschiedene Möglichkeiten denkbar, um auf die Abweichung des Kennwertes von seinem Sollwert zu reagieren: Der Einsatz chemischer Hilfsstoffe, Änderung der Faserbehandlung oder -zusammensetzung oder Versteifen der Papiermaschine bis zur Herabsetzung der Siebgeschwindigkeit.

Die Zugabemenge von festigkeitserhöhenden Chemikalien und/oder Retentionsmitteln zur Faserstoffsuspension kann so eingestellt werden, dass ein Kennwert eingehalten wird, der als zweckmäßig festgelegt wurde. Mit Vorteil kann ihre Zugabemenge gemäß den Anforderungen an die Runnability minimiert werden.

Mit Hilfe des erfindungsgemäßen Verfahrens kann der Papiermaschinen-Prozess einfach und wirtschaftlich auf die optimale Runnability auf der Papiermaschine gebracht werden. Es ermöglicht nämlich eine Korrektur des Bindekräfte-Potentials, und zwar rechtzeitig, also bevor auf der Papiermaschine ein Abriss erfolgt ist. Die Verhinderung von Abrissen ist bekanntlich ein ganz entscheidender wirtschaftlicher Vorteil bei der Papiererzeugung.

Die Erfindung und ihre Vorteile wird an Hand einer Zeichnung erläutert.

Gemäß dieser Zeichnung wird der bereits aufbereitete Faserstoff 1 mit dem Verdünnungswasser 2, das z.B. aus dem Siebwasser der Papiermaschine stammt, in der Mischpumpe 3 vermischt. Dadurch bildet sich eine Faserstoffsuspension, welche die Stoffdichte hat, die für den Betrieb auf der Papiermaschine vorgesehen ist. Diese Faserstoffsuspension gelangt bei dem hier gezeigten Beispiel über eine Steigleitung 5 zuerst in eine Cleanerbatterie 8 und dann in einen Entlüftungsbehälter 9. Die Steigleitung 5 bringt die Fasersuspension auf ein höheres geodätisches Niveau, was die Entlüftung durch Unterdruck erleichtert. Bekanntlich dient die Cleanerbatterie 8 zum Ausscheiden feiner Schwerteile und der Entlüftungsbehälter 9, um mit Hilfe von Unterdruck die Luft 10 aus der Suspension zu entfemen. Solche und ähnliche Behandlungsschritte sind an sich bekannt und daher ist nur diese eine Möglichkeit schematisch gezeigt. Im Zusammenhang mit dem erfindungsgemäßen Verfahren ist es von Vorteil, wenn die Messstelle 4, an der die Luftgehaltsmessung vorgenommen wird, dort liegt, wo die Entlüftung der Faserstoffsuspension noch nicht durchgeführt wurde. Aus der Messstelle 4 wird über eine Signalleitung 6 ein Regler 7 angesteuert, der die bereits beschriebenen Korrekturen oder Regelvorgänge veranlassen kann. Das kann, wie hier exemplarisch dargestellt ist, die Steuerung einer Dosierpumpe 14 sein, mit der ein chemisches Retentionsmittel 15 in die Suspension zugegeben wird. Alternativ sind in dieser Figur zwei weitere Messstellen angedeutet, bei denen die Messung an dem noch nicht verdünnten Faserstoff 1 erfolgt, z.B. in der Ablaufleitung der entsprechenden Bütte 13 (Messstelle 4') oder an einer aus der Bütte 13 genommenen Stoffprobe (Messstelle 4").

Die gereinigte und entlüftete Fasersuspension wird durch eine Fallleitung 11 zur Pumpe 12 geführt, aus der sie z.B. in die Stoffzentrale oder in den Stoffauflauf einer Papiermaschine gelangen kann. Die Stoffzentrale dient z.B. der Vermischung von verschiedenen zur Papiererzeugung verwendeten Komponenten.

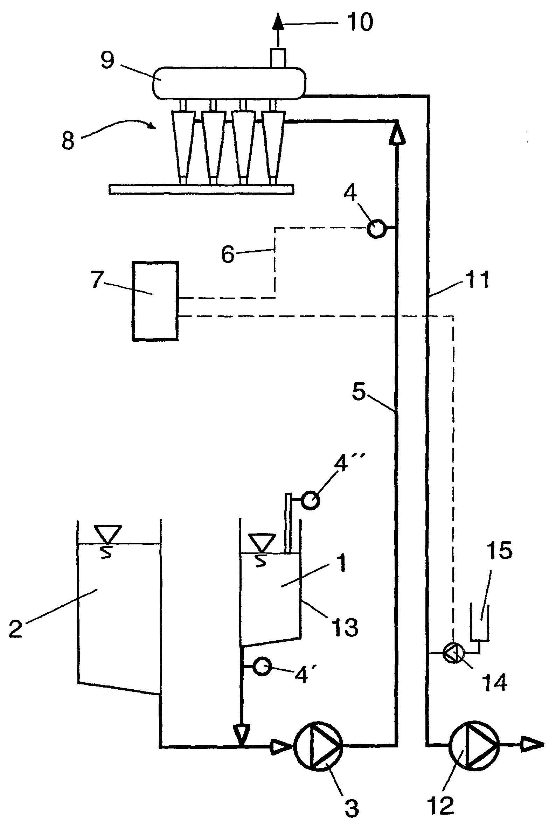

## Patentansprüche

1. Verfahren zur Bestimmung eines Kennwertes für das Bindekräfte-Potential von Papierfasem in einer zur Papiererzeugung aufbereiteten Fasersuspension,
**dadurch gekennzeichnet,**
**dass** der Luftgehalt der Fasersuspension gemessen und dass daraus der Kennwert abgeleitet wird.

2. Verfahren nach Anspruch 1,
**dadurch gekennzeichnet,**
**dass** der Luftgehalt an einer Messstelle (4, 4', 4") im Faserstoff-Aufbereitungsprozess gemessen wird, an der die Fasersuspension die für die Papiererzeugung geforderte Stoffzusammensetzung hat, ohne dass der Luftgehalt durch einen entsprechenden Entlüftungsprozess eingestellt wurde.

3. Verfahren nach Anspruch 2,
**dadurch gekennzeichnet,**
**dass** der Luftgehalt im Konstanten Teil einer Papierfabrik nach der Mischpumpe (3) gemessen wird, in der der Faserstoff (1) mit Verdünnungswasser (2) vermischt wird.

4. Verfahren nach Anspruch 2,
**dadurch gekennzeichnet,**
**dass** der Luftgehalt im Konstanten Teil einer Papierfabrik vor der Mischpumpe (3) gemessen wird, in der der Faserstoff (1) mit Verdünnungswasser (2) vermischt wird.

5. Verfahren nach Anspruch 2, 3 oder 4,
**dadurch gekennzeichnet,**
**dass** die Fasersuspension nach Messung des Luftgehaltes durch Unterdruck entlüftet wird.

6. Verfahren nach einem der voranstehenden Ansprüche,
**dadurch gekennzeichnet,**
**dass** mehrere Faserstoffsorten gleichzeitig zur Papiererzeugung eingesetzt werden und dass der Luftgehalt bei den Sorten getrennt gemessen wird.

7. Verfahren nach einem der voranstehenden Ansprüche,
**dadurch gekennzeichnet,**
**dass** der Kennwert für das Bindekräfte-Potential mit Hilfe von entsprechenden Erfahrungswerten dem Luftgehalt zugeordnet wird.

8. Verfahren nach Anspruch 7,
**dadurch gekennzeichnet,**
**dass** die Erfahrungswerte durch Messung der Festigkeiten der aus der Suspension gebildeten Blätter ermittelt werden.

9. Verfahren nach Anspruch 7,
**dadurch gekennzeichnet,**
**dass** die Erfahrungswerte durch Ermittlung der mit der Fasersuspension erzielten Runnability auf der Papiermaschine zugeordnet werden.

10. Verwendung des Verfahrens nach einem der voranstehenden Ansprüche,
**dadurch gekennzeichnet,**
**dass** die Zugabemenge von festigkeitserhöhenden Chemikalien und/oder Retentionsmitteln zur Fasersuspension so eingestellt wird, dass ein Kennwert eingehalten wird, der als zweckmäßig festgelegt wurde.

11. Verwendung des Verfahrens nach einem der voranstehenden Ansprüche,
**dadurch gekennzeichnet,**
**dass** die Zugabemenge von festigkeitserhöhenden Chemikalien und/oder Retentionsmitteln zur Fasersuspension gemäß den Anforderungen an die Runnability minimiert wird.

## Claims

1. Method for determining a characteristic value for the binding strength of paper fibres in a fibre suspension prepared for the production of paper,
**characterised in that**
the air content of the fibre suspension is measured, and **in that** the characteristic value is derived from this.

2. Method as in Claim 1,
**characterised in that**
the air content is measured at a measuring point (4, 4', 4") in the pulp preparation process at which the fibre suspension has the material composition required for the production of paper, without the air content having been adjusted by means of a suitable de-airing process.

3. Method as in Claim 2,
**characterised in that**
the air content is measured in the constant part of a paper factory, after the mixing pump (3) in which the pulp (1) is mixed with dilution water (2).

4. Method as in Claim 2,
**characterised in that**
the air content is measured in the constant part of a paper factory, before the mixing pump (3) in which the pulp (1) is mixed with dilution water (2).

5. Method as in Claim 2, 3 or 4,
**characterised in that**,
after the air content has been measured, the fibre suspension is de-aired by applying a partial vacuum.

6. Method as in one of the preceding Claims,
**characterised in that**
a number of types of pulp are used at the same time to produce paper, and **in that** the air content of the types is measured separately.

7. Method as in one of the preceding Claims,
**characterised in that**
the characteristic value for the binding strength potential is assigned to the air content with the aid of appropriate empirical values.

8. Method as in Claim 7,
**characterised in that**
the empirical values are established by measuring the strength levels of sheets made from the suspension.

9. Method as in Claim 7,
**characterised in that**
the empirical values are assigned by establishing the runability on the paper machine achieved by the fibre suspension.

10. Use of the method as in one of the preceding Claims,
**characterised in that**
the quantities added to the fibre suspension of chemicals to increase strength and/or of retention agents are adjusted so that a characteristic value which has been found to be suitable is maintained.

11. Use of the method as in one of the preceding Claims,
**characterised in that**
the quantities added to the fibre suspension of chemicals to increase strength and/or of retention agents are minimised in accordance with the requirements for runability.

## Revendications

1. Procédé destiné à la détermination d'une valeur caractéristique pour le potentiel des forces de liaison de fibres de papier dans une suspension fibreuse traitée pour la fabrication de papier,
**caractérisé en ce que**
la teneur en air de la suspension fibreuse est mesurée, et **en ce que** la valeur caractéristique est dérivée de cette mesure.

2. Procédé selon la revendication 1,
**caractérisé en ce que**
la teneur en air est mesurée dans le processus de traitement de la matière fibreuse au niveau d'un point de mesure (4, 4', 4"), au niveau duquel la composition de la suspension fibreuse est celle exigée pour la fabrication de papier, sans que la teneur en air ait été réglée par un processus de désaération correspondant.

3. Procédé selon la revendication 2,
**caractérisé en ce que**
la teneur en air est mesurée dans la partie constante d'une usine à papier en aval de la pompe mélangeuse (3) , dans laquelle la matière fibreuse (1) est mélangée avec de l'eau dè dilution (2).

4. Procédé selon la revendication 2,
**caractérisé en ce que**
la teneur en air est mesurée dans la partie constante d'une usine à papier en amont de la pompe mélangeuse (3), dans laquelle la matière fibreuse (1) est mélangée avec de l'eau de dilution (2).

5. Procédé selon la revendication 2, 3 ou 4,
**caractérisé en ce que,**
après la mesure de la teneur en air, la suspension fibreuse est désaérée par dépression.

6. Procédé selon l'une des revendications précédentes,
**caractérisé en ce que**
plusieurs sortes de matières fibreuses sont utilisées simultanément pour la fabrication de papier, et **en ce que** la teneur en air des sortes est mesurée séparément.

7. Procédé selon l'une des revendications précédentes,
**caractérisé en ce que**
la valeur caractéristique pour le potentiel des forces de liaison est associée à la teneur en air à l'aide de valeurs empiriques correspondantes.

8. Procédé selon la revendication 7,
**caractérisé en ce que**
les valeurs empiriques sont déterminées par la mesure des résistances des feuilles engendrées à partir de la suspension.

9. procédé selon la revendication 7,
**caractérisé en ce que**
les valeurs empiriques sont associées par détermination du comportement obtenu avec la suspension fibreuse sur la machine à papier.

10. Procédé selon l'une des revendications précédentes,
**caractérisé en ce que**
la quantité de produits chimiques et/ou d'agents de rétention augmentant la résistance ajoutée à la suspension fibreuse est dosée de façon à respecter une valeur caractéristique ayant été déterminée comme étant appropriée.

11. Procédé selon l'une des revendications précédentes,
**caractérisé en ce que** la quantité de produits chimiques et/ou d'agents de rétention augmentant la résistance ajoutée à la suspension fibreuse est minimisée en fonction des exigences du comportement.
